# EUROPEAN PATENT SPECIFICATION

(11) **EP 1 514 508 B1**
(45) Date of publication and mention of the grant of the patent: **04.07.2007**
(21) Application number: 04021461.1
(22) Date of filing: 09.09.2004
(51) Int. Cl.: A61B 1/005, A61M 25/00

(54) **Flexible tube of endoscope and method for manufacturing the same**
Flexibler Schlauch eines Endoskops und Verfahren zu seiner Herstellung
Tube flexible d'une endoscope et procédé de sa fabrication

(30) Priority: 11.09.2003 JP 2003320286
(43) Date of publication of application: 16.03.2005
(73) Proprietor: Olympus Corporation, Tokyo 151-0072 (JP)
(72) Inventor: Nishiie, Takehiro, Hachioji, Tokyo (JP)
(74) Representative: von Hellfeld, Axel

(56) References cited:
- WO-A-01/23022
- WO-A-20/04037075
- DE-A- 3 910 746
- US-A- 4 495 134
- US-A- 5 083 549
- PATENT ABSTRACTS OF JAPAN vol. 2000, no. 24, 11 May 2001 (2001-05-11) & JP 2001 190494 A (ASAHI OPTICAL CO LTD), 17 July 2001 (2001-07-17)
- PATENT ABSTRACTS OF JAPAN vol. 1998, no. 12, 31 October 1998 (1998-10-31) & JP 10 179509 A (OLYMPUS OPTICAL CO LTD), 7 July 1998 (1998-07-07)

## Description

### BACKGROUND OF THE INVENTION

### 1. Field of the Invention

The present invention relates to a flexible tube of an endoscope used in the fields of medical care, industries, and the like and to a method for manufacturing the same.

### 2. Description of the Related Art

In recent years, endoscopes have been widely used for the intracavitary inspection and diagnosis, the inspection of the inside of a plant, and the like in the medical field and the industrial field. In general, a flexible tube of an endoscope has the configuration in which the outer surface of a helical tube is covered with a net-shaped tube and, furthermore, the outer surface of the net-shaped tube is covered with an outer sheath resin.

With respect to these known flexible tubes of endoscopes, the compounding ratio of the outer sheath resin is varied, or an outer sheath resin is formed into the shape of a taper, as disclosed in Japanese Unexamined Patent Application Publication No. 2001-190494, for example.

However, it is difficult to ensure an increased length of the above-described portion, in which the compounding ratio of the outer sheath resin is varied, or the taper portion disclosed in the above-described document. Consequently, it is difficult to increase the flexibility difference between the softest portion and the hardest portion and, in addition, to manufacture a flexible tube having a gentle gradient of the flexibility variation.

With respect to the molding of the tapered shape, previously, the molding is performed by varying the passing speed of a flexible tube passing through a die for extrusion. However, this method requires enormous efforts to optimally satisfy all the length and the start position of the taper portion, the difference in outer diameters between the small diameter portion and the large diameter portion, and the adhesion between the net-shaped tube and the resin.

United States Patent No. 4,495,134 describes a flexible tube for use in an endoscope having a flexibility which varies in a step-wise manner, i.e. with step discontinuities between regions of different flexibilities, from one end of the tube to the other.

The present invention has been made in consideration of the above-described circumstances. Accordingly, it is an object of the present invention to provide a flexible tube of an endoscope which can be manufactured easily and in which a large flexibility difference between the softest portion and the hardest portion can be ensured and, in addition, the gradient of the flexibility variation can be made smooth, and to provide a method for manufacturing the same.

### SUMMARY OF THE INVENTION

The present invention provides a flexible tube of an endoscope having the features as recited in claim 1. Preferred features of the present invention are recited in the dependent claims.

A flexible tube of an endoscope according to the present invention is provided with a first flexibility-varying portion disposed in an outer sheath of a flexible tube having a distal end and a base end, while the thickness of the outer sheath in the distal end side of the first flexibility-varying portion is varied from the thickness in the base end side and the flexibility of the flexible tube in the distal end side is varied from the flexibility in the base end side. A second flexibility-varying portion is disposed in the outer sheath, at a location different from that of the first flexibility-varying portion in a longitudinal direction of the flexible tube, while the second flexibility-varying portion is disposed substantially without varying the thickness of the outer sheath in the longitudinal direction of the flexible tube and the flexibility of the flexible tube in the distal end side is varied from the flexibility in the base end side.

In a method for manufacturing a flexible tube of an endoscope according to the present invention, a first covering layer is formed on a member formed substantially in the shape of a long-length tube having a distal end and a base end, while the first covering layer includes a flexibility-varying portion which varies the flexibility of the flexible tube in a longitudinal direction substantially without varying the thickness. Subsequently, a second covering layer is formed on the outside of the first covering layer, while the second covering layer includes a flexibility-varying portion which varies the flexibility of the flexible tube in the longitudinal direction by varying the thickness from the distal end side toward the base end side of the flexible tube. Thereafter, the first covering layer and the second covering layer are brought into intimate contact with each other.

According to the flexible tube of the endoscope of the present invention and the method for manufacturing the same, such excellent effects are exerted that the manufacture is performed easily, a large flexibility difference between the softest portion and the hardest portion can be ensured and, in addition, the gradient of the flexibility variation can be made smooth.

The above features and advantages of the invention will become more clearly understood from the following description referring to the accompanying drawings.

### BRIEF DESCRIPTION OF THE DRAWINGS

Fig. 1 is a schematic configuration diagram showing the entire electronic endoscope, according to a first embodiment of the present invention.
Fig. 2 is a schematic configuration diagram showing the internal structure of a flexible tube, according to the first embodiment of the present invention.
Fig. 3A is a sectional view of the flexible tube, according to the first embodiment of the present invention.
Fig. 3B is an explanatory diagram of the flexibility of the flexible tube, according to the first embodiment of the present invention.
Fig. 4A is an explanatory diagram of a step of applying a covering of an outer sheath resin serving as the second layer of the flexible tube, according to the first embodiment of the present invention.
Fig. 4B is an explanatory diagram of the state in which a heat-shrinkable tube in the state shown in Fig. 4A has been peeled off.
Fig. 5A is an explanatory diagram of the insertion of the stepped flexible tube shown in Fig. 4B into a grinder.
Fig. 5B is an explanatory diagram showing the state of the stepped flexible tube shown in Fig. 4B after being ground with the grinder.
Fig. 6A is a sectional view of a flexible tube, according to a second embodiment of the present invention.
Fig. 6B is an explanatory diagram of the flexibility of the flexible tube, according to the second embodiment of the present invention.
Fig. 7A is a sectional view of a flexible tube, according to a third embodiment of the present invention.
Fig. 7B is an explanatory diagram of the flexibility of the flexible tube, according to the third embodiment of the present invention.

### DETAILED DESCRIPTION OF THE PREFERRED EMBODIMENTS

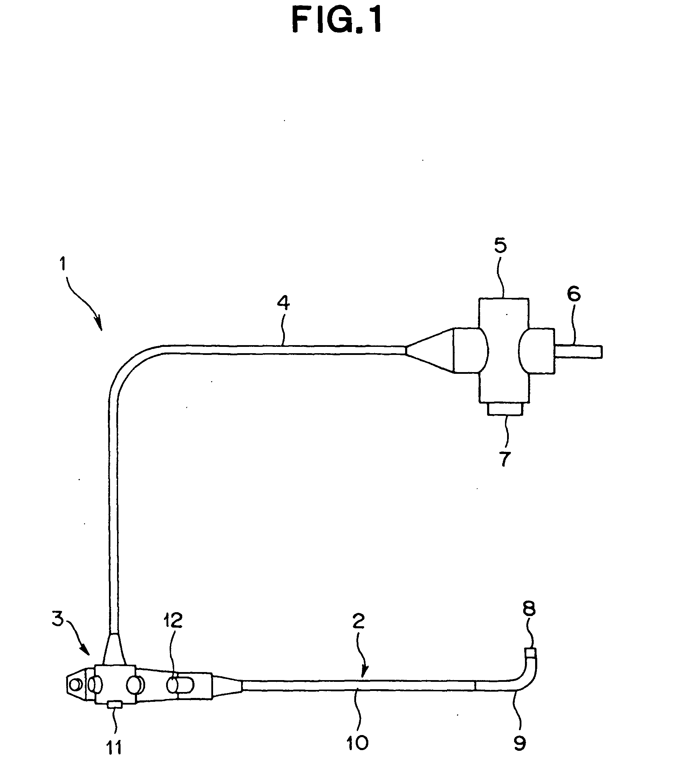

A first embodiment of the present invention will be described below with reference to Fig. 1 to Fig. 5B.

In Fig. 1, reference numeral 1 denotes an electronic endoscope adopted in the first embodiment. The electronic endoscope 1 includes a slender insertion portion 2, a control portion 3 which is connected to a proximal end side of the insertion portion 2 and which is grasped by a surgeon to perform various operations, and a universal cord 4 which is disposed extending from the control portion 3.

At the other end of the universal cord 4, a connector portion 5 connected to a light source and a camera control unit (hereafter abbreviated as CCU"), although not shown in the drawing, is disposed. In this case, a light guide connector 6 is connected to the light source, and a camera connector 7 is connected to the CCU.

The insertion portion 2 includes a flexible tube 10 having flexibility, joined to the control portion 3, a bending portion 9 joined to the distal end of the flexible tube 10, and a distal end 8 joined to the distal end of the bending portion 9. On the other hand, the control portion 3 is provided with, for example, a bending control lever 11 for controlling the bending of the bending portion 9 in the vertical and horizontal direction and a therapeutic instrument insertion hole 12 for inserting a therapeutic instrument, e.g., forceps.

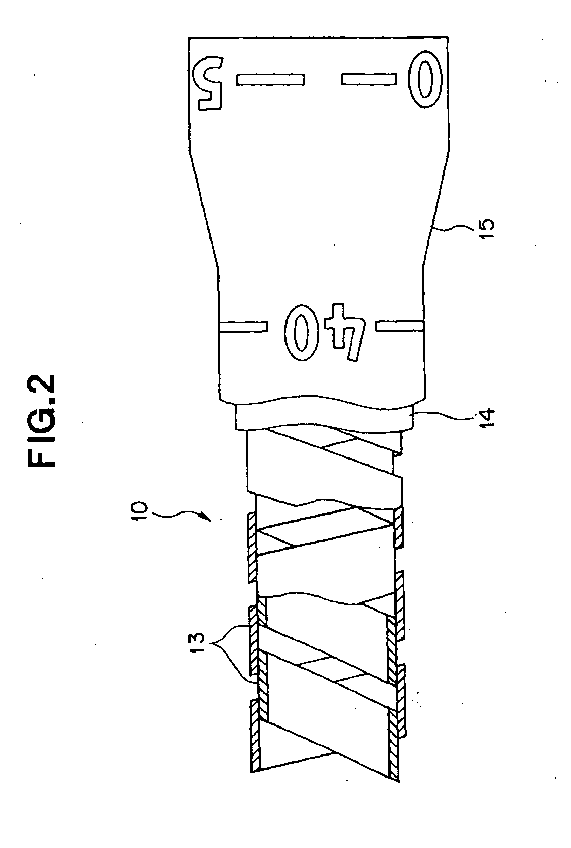

As shown in Fig. 2, the flexible tube 10 is primarily composed of a helical tube 13 in which two pieces of metal band are wound helically, a net-shaped tube 14 formed into the shape of a net covering the helical tube 13, and an outer sheath resin 15 covering the outside of the net-shaped tube 14 in that order from the inside. The outer sheath resin 15 is formed into the shape of a taper at some midpoint of the flexible tube 10, while the taper has a diameter increasing from the distal end side toward the base end side, as is clear from the appearance thereof.

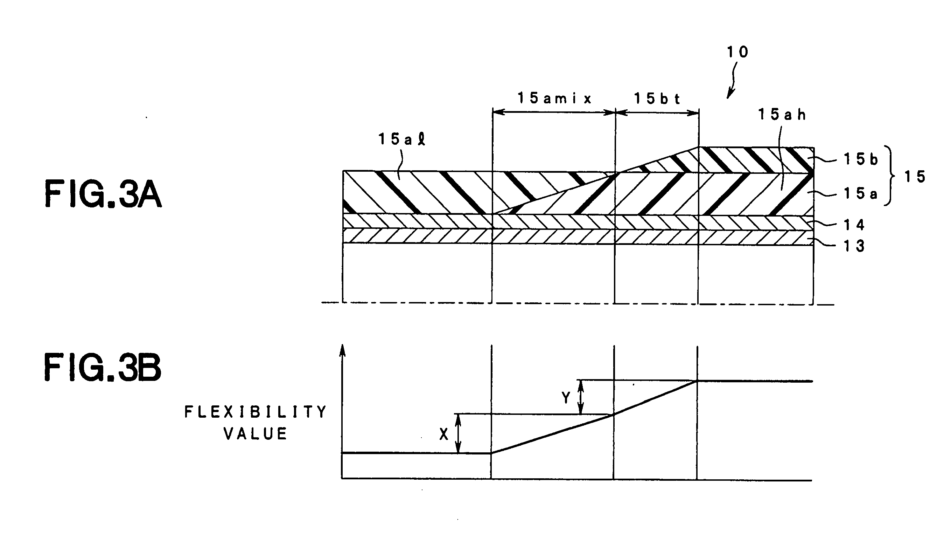

As shown in Fig. 3A, the outer sheath resin 15 of the flexible tube 10 is composed of a plurality of layers, incidentally, is composed of two layers in the first embodiment. An outer sheath resin 15a, which is the first layer in the net-shaped tube 14 side of the outer sheath resin 15, is formed by compounding a soft resin as a first resin (distal end side) and a hard resin as a second resin (base end side) (in Fig. 3A, reference numeral 15al denotes a soft resin portion of the outer sheath resin 15a serving as the first layer and reference numeral 15ah denotes a hard resin portion of the outer sheath resin 15a serving as the first layer).

Specifically, in the outer sheath resin 15a serving as the first layer, the portion from the distal end of the flexible tube to the position at a distance of about 200 mm from the distal end (an index of about 30 when expressed in the index of flexible tube, where the index of flexible tube refers to a distance from the distal end of an endoscope) is formed of the soft resin portion 15al. With respect to the outer sheath resin 15a serving as the first layer, in the portion from the position at a distance of about 200 mm from the distal end of the flexible tube to the position at a distance of about 300 mm (an index of about 40 in terms of index of flexible tube), the soft resin portion 15al is gradually replaced with the hard resin portion 15ah, and this portion constitutes a resin-hardness-varying portion 15amix serving as the second flexibility-varying portion. Here, with respect to the resin materials for the outer sheath resin 15a serving as the first layer, for example, the soft resin portion 15al is composed of an ester-based resin, and the hard resin portion 15ah is composed of the same-ester-based but harder resin.

In an outer sheath resin 15b serving as the second layer which is an outermost layer, while the outer sheath resin 15b is disposed outside the outer sheath resin 15a serving as the first layer of the outer sheath resin 15, a taper portion 15bt serving as a first flexibility-varying portion is disposed. The taper portion 15bt has a wall thickness gradually increasing from the position substantially following the base end side of the resin-hardness-varying portion 15amix of the outer sheath resin 15a serving as the first layer toward the base end side is disposed. Here, the same ester-based resin as that used for the soft resin portion 15al of the outer sheath resin 15a serving as the first layer is used as the resin material for the outer sheath resin 15b serving as the second layer.

Steps for manufacturing the above-described flexible tube 10 will be described below.

In the first step, as in known flexible tubes, a covering of the outer sheath resin 15a serving as the first layer composed of a soft resin and a hard resin is applied. As described above, in the outer sheath resin 15a serving as the first layer, the portion from the distal end of the flexible tube to the position at a distance of about 200 mm from the distal end is formed of the soft resin portion 15al. In the portion from this position at a distance of about 200 mm from the distal end of the flexible tube to the position at a distance of about 300 mm, the soft resin portion 15al is gradually replaced with the hard resin portion 15ah, and this portion constitutes a resin-hardness-varying portion 15amix.

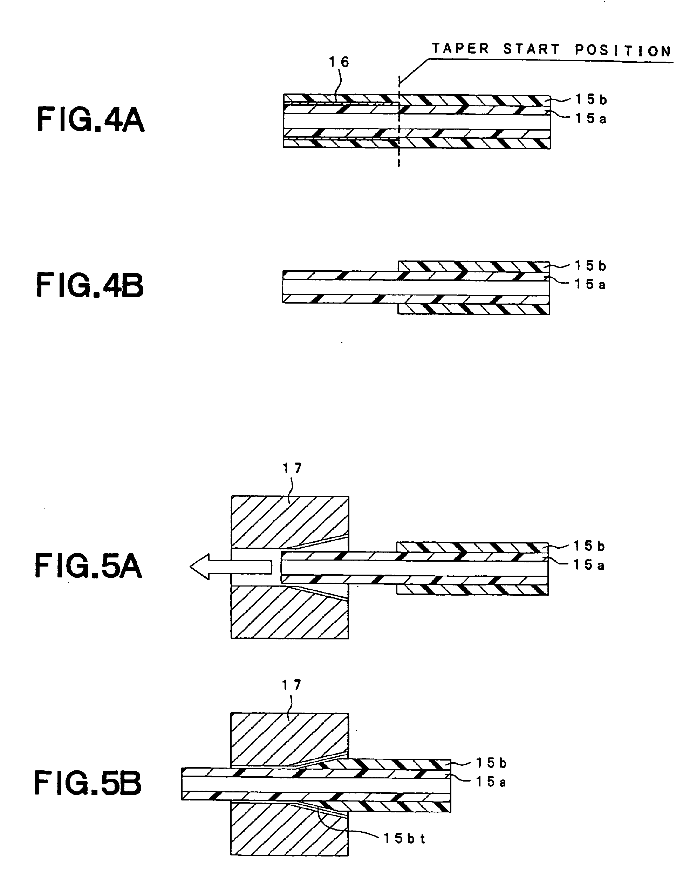

In the second step, the outer sheath resin 15a serving as the first layer is covered with a heat-shrinkable tube 16, up to the location in the neighborhood of the position at which the replacement of the soft resin portion 15al with the hard resin portion 15ah is completed (in the neighborhood of the position indicated by an index of about 40), that is, up to the start position of the taper portion 15bt at which the resin-hardness-varying portion 15amix terminates, and under this condition, a covering of the outer sheath resin 15b serving as the second layer is applied (Fig. 4A).

In the third step, the outer sheath resin 15b serving as the second layer located in the side nearer to the distal end than is the taper start position is peeled off together with the heat-shrinkable tube 16, so that a stepped flexible tube shown in Fig. 4B is prepared.

In the forth step, as shown in Fig. 5A and Fig. 5B, the taper portion 15bt is formed from the stepped flexible tube prepared in the third step by the use of a grinder 17. The grinding may be performed either by rotation of the grindstone side of the grinder 17 or by rotation of the stepped flexible tube. Alternatively, a high-heat mold or the like which partially fuses the taper portion 15bt may be used in place of the grinder 17.

In the fifth step, the net-shaped tube 14 and the outer sheath resin 15a serving as the first layer are joined by heat adhesion, and likewise, the outer sheath resin 15a serving as the first layer and the outer sheath resin 15b serving as the second layer are joined by heat adhesion. At this time, it is desirable that the heat adhesion is performed at 180°C to 220°C for 10 to 15 minutes, for example. However, the heat-adhesion condition is not limited to this as long as an optimum condition is set based on the melting points and the heat resistance of the outer sheath resin 15a serving as the first layer and the outer sheath resin 15b serving as the second layer.

In the sixth step, the white line ink and the top coat are applied as in known flexible tubes.

With respect to the thus prepared flexible tube 10 according to the first embodiment of the present invention, by dividing the manufacturing process into a step of ensuring the adhesion between the net-shaped tube 14 and the outer sheath resin 15a serving as the first layer and between the outer sheath resin 15a serving as the first layer and the outer sheath resin 15b serving as the second layer (in particular, the fifth step), a step of applying the coverings of the outer sheath resins 15a and 15b in which it is essential only that the outer diameters of the small diameter portion and the large diameter portion of the flexible tube 10 are satisfied (in particular, the first and the second steps), and a step of forming the taper portion 15bt in which it is essential only that the length and the start position of the taper portion 15bt of the outer sheath resin 15b serving as the second layer are satisfied (in particular, the third and the fourth steps), determination of each optimum condition becomes easy, and the flexible tube 10 is manufactured optimally and easily.

Since no adhesive is required in the fifth step of joining by the heat adhesion, fluctuations in the adhesion due to variations in the adhesive are reduced. In particular, in the neighborhood of the start position of the taper portion 15bt, no adhesive extends off the portion where the outer sheath resin 15b serving as the second layer is disposed, nor occurs, conversely, shortage of the adhesive in the neighborhood of the start position of the taper portion 15bt.

With respect to the flexible tube 10 according to the first embodiment of the present invention, as shown in Fig. 3B, initially, smooth flexibility variation as in known flexible tubes is achieved by the resin-hardness-varying portion 15amix of the outer sheath resin 15a serving as the first layer (a portion X in Fig. 3B). Subsequently, the flexibility variation due to the taper portion 15bt of the outer sheath resin 15b serving as the second layer is achieved substantially following the resin-hardness-varying portion 15amix (a portion Y in Fig. 3B). In this manner, in the entire flexible tube 10, the large flexibility variation can be achieved with a gentle, long gradient.

In the above-described flexible tube 10, a larger load is applied to the outer sheath resin 15b serving as the second layer when the flexible tube 10 is bended repeatedly. This is because larger distortion due to the bending occurs in the outer sheath resin 15b serving as the second layer located farther from the center line of the flexible tube 10. However, the bending resistance can be improved by using a soft resin for the outer sheath resin 15b serving as the second layer.

Further, various specifications are made for the desired flexible tube 10. However, the flexible tube 10 according to the first embodiment of the present invention can be flexibly complied with various specifications required for the flexibility variation by combining the shapes of the outer sheath resin 15a serving as the first layer, the outer sheath resin 15b serving as the second layer, and the taper portion 15bt and a compounding ratio of each resin. For example, with respect to the taper portion 15bt of the outer sheath resin 15b serving as the second layer, a softer resin provides gentler flexibility variation even when the shapes of the taper portions are the same. Conversely, a harder resin provides a larger amount of flexibility variation. With respect to the taper portion 15bt, even when the resins are the same, if the gradient of the taper shape were gentler, the flexibility variation would become gentler, and if the gradient of the taper shape were steeper, the amount of flexibility variation would become larger.

The flexible tube 10 according to the first embodiment of the present invention is described with reference to the example in which the ester-based resin is used as the resin material for the soft resin portion 15al of the outer sheath resin 15a serving as the first layer, and the same-ester-based but harder resin is used for the hard resin portion 15ah. The description is made with reference to the example in which the same ester-based resin as that used for the soft resin portion 15al of the outer sheath resin 15a serving as the first layer is used as the resin material for the outer sheath resin 15b serving as the second layer. However, these resin materials are not limited thereto, and resins of, for example, ester-based, olefin-based, styrene-based, and amide-based may be used alone or after blending for the outer sheath resins serving as respective layers in accordance with the required flexibility.

The flexible tube 10 according to the first embodiment of the present invention has a configuration in which the resin-hardness-varying portion 15amix of the outer sheath resin 15a serving as the first layer is substantially followed by the taper portion 15bt of the outer sheath resin 15b serving as the second layer. However, these are not necessarily continuous. For example, some distance may be provided between the resin-hardness-varying portion 15amix of the outer sheath resin 15a serving as the first layer and the taper portion 15bt of the outer sheath resin 15b serving as the second layer, or the two may overlap with each other to some extent.

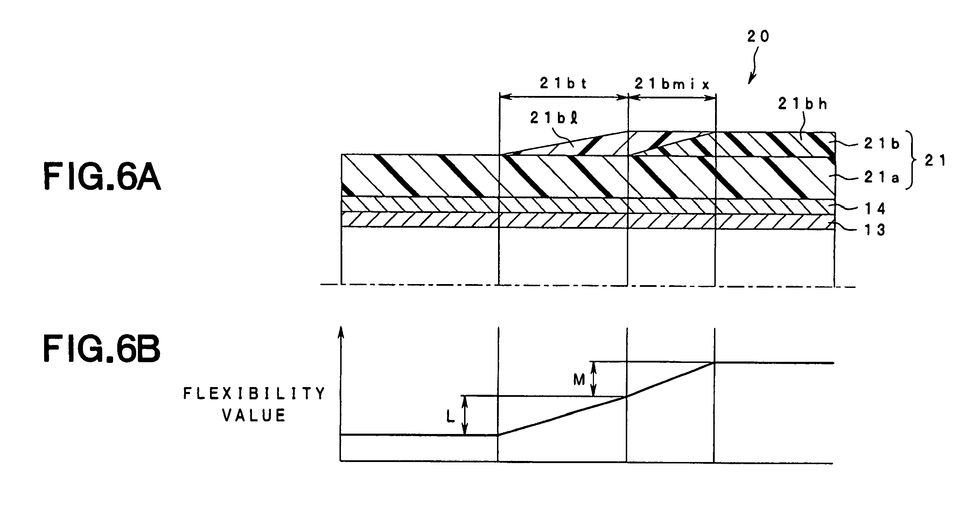

A second embodiment of the present invention will be described below with reference to Fig. 6A and Fig. 6B.

In the second embodiment, in contrast to the above-described first embodiment, both the resin-hardness-varying portion serving as the flexibility-varying portion to vary the flexibility, and the taper portion are disposed in the outer sheath resin serving as the second layer. The other configurations are similar to those in the first embodiment and, therefore, the explanation thereof will not be provided.

That is, in Fig. 6A, reference numeral 20 denotes a flexible tube. The flexible tube 20 is primarily composed of a helical tube 13, a net-shaped tube 14 formed to have the shape of a net covering the helical tube 13, and an outer sheath resin 21 covering the outside of the net-shaped tube 14 in that order from the inside. The outer sheath resin 21 is formed into the shape of a taper at some midpoint of the flexible tube 20, while the taper has a wall thickness increasing from the distal end side toward the base end side, as is clear from the appearance thereof.

The outer sheath resin 21 of the flexible tube 20 is composed of a plurality of layers, incidentally, is composed of two layers in the second embodiment. An outer sheath resin 21a, which is the first layer in the net-shaped tube 14 side of the outer sheath resin 21, is formed of one type of soft resin, for example.

In an outer sheath resin 21b serving as the second layer which is disposed outside the outer sheath resin 21a serving as the first layer of the outer sheath resin 21, a taper portion 21bt serving as a first flexibility-varying portion is disposed in the distal end side while having a wall thickness gradually increasing toward the base end side. The outer sheath resin 21b serving as the second layer is formed by compounding a soft resin as a first resin (distal end side) and a hard resin as a second resin (base end side) (in Fig. 6A, reference numeral 21bl denotes a soft resin portion of the outer sheath resin 21b serving as the second layer and reference numeral 21bh denotes a hard resin portion of the outer sheath resin 21b serving as the second layer).

Specifically, the outer sheath resin 21b serving as the second layer is formed of the soft resin portion 21bl up to the position in the neighborhood of the position where the taper portion 21bt terminates. In the outer sheath resin 21b serving as the second layer, a resin-hardness-varying portion 21bmix serving as the second flexibility-varying portion is formed, in which the soft resin portion 21bl is gradually replaced with the hard resin portion 21bh from the position in the neighborhood of the position where the taper portion 21bt of the soft resin portion 21bl terminates.

Here, an ester-based resin, for example, is used as the resin material for the outer sheath resin 21a serving as the first layer. With respect to the rein material for the outer sheath resin 21b serving as the second layer, for example, the same ester-based resin as that used for the outer sheath resin 21a serving as the first layer is used for the soft resin portion 21bl, and the same-ester-based but harder resin is used for the hard resin portion 21bh. However, selection of these resin materials is not limited thereto, and resins of, for example, ester-based, olefin-based, styrene-based, and amide-based may be used alone or after blending for the outer sheath resins serving as respective layers in accordance with the required flexibility.

With respect to the thus prepared flexible tube 20 according to the second embodiment of the present invention, as in the above-described first embodiment, by dividing the manufacturing process into a step of ensuring the adhesion between the net-shaped tube 14 and the outer sheath resin 21a serving as the first layer and between the outer sheath resin 21a serving as the first layer and the outer sheath resin 21b serving as the second layer, a step of applying the coverings of the outer sheath resins 21a and 21b in which it is essential only that the outer diameters of the small diameter portion and the large diameter portion of the flexible tube 20 are satisfied, and a step of forming the taper portion 21bt in which it is essential only that the length and the start position of the taper portion 21bt of the outer sheath resin 21b serving as the second layer are satisfied, determination of each optimum condition becomes easy, and the flexible tube 20 can be manufactured optimally and easily.

Since no adhesive is required in the joining by the heat adhesion as in the above-described first embodiment, fluctuations in the adhesion due to variations in the adhesive are reduced. In particular, in the neighborhood of the start position of the taper portion 21bt, no adhesive extends off the portion where the outer sheath resin 21b serving as the second layer is disposed, nor occurs, conversely, shortage of the adhesive in the neighborhood of the start position of the taper portion 21bt.

Further, with respect to the flexible tube 20 according to the second embodiment of the present invention, as shown in Fig. 6B, initially, smooth flexibility variation as in known flexible tubes is achieved by the taper portion 21bt of the outer sheath resin 21b serving as the second layer (a portion L in Fig. 6B). Subsequently, the flexibility variation due to the resin-hardness-varying portion 21bmix of the outer sheath resin 21b serving as the second layer is achieved substantially following the taper portion 21bt (a portion M in Fig. 6B). In this manner, in the entire flexible tube 20, the large flexibility variation can be achieved with a gentle, long gradient.

In the above-described flexible tube 20, a larger load is applied to the outer sheath resin 21b serving as the second layer when the flexible tube 20 is bended repeatedly. This is because larger distortion due to the bending occurs in the outer sheath resin 21b serving as the second layer located farther from the center line of the flexible tube 20. However, the bending resistance can be improved by using a soft resin for the outer sheath resin 21b serving as the second layer.

Further, various specifications are made for the desired flexible tube 20. However, the flexible tube 20 according to the second embodiment of the present invention can be flexibly complied with various specifications required for the flexibility variation by combining the shapes of the outer sheath resin 21a serving as the first layer, the outer sheath resin 21b serving as the second layer, and the taper portion 21bt and a compounding ratio of each resin.

The flexible tube 20 according to the second embodiment of the present invention has a configuration in which the taper portion 21bt of the outer sheath resin 21b serving as the second layer is substantially followed by the resin-hardness-varying portion 21bmix. However, these are not necessarily continuous. For example, some distance may be provided between the taper portion 21bt and the resin-hardness-varying portion 21bmix, or the two may overlap with each other to some extent.

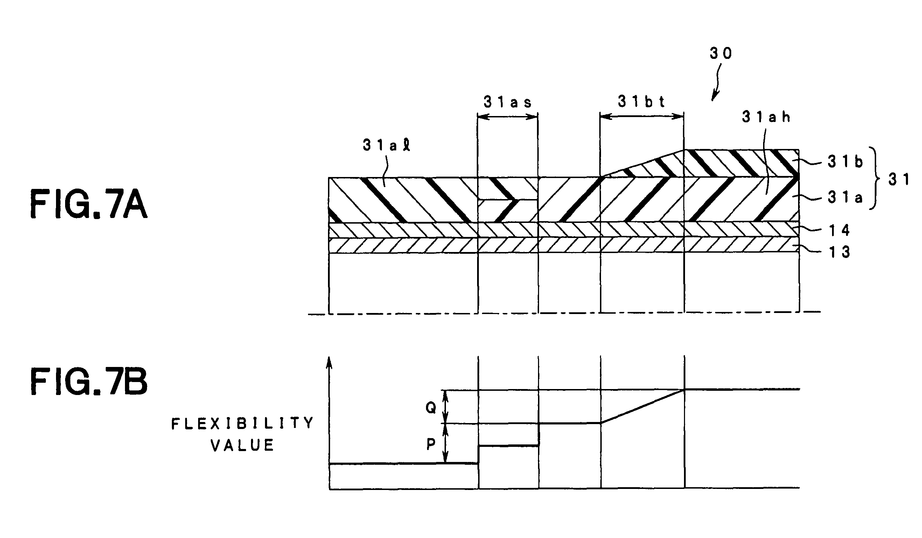

A third embodiment of the present invention will be described below with reference to Fig. 7A and Fig. 7B.

In the third embodiment, in contrast to the above-described first embodiment, the outer sheath resin serving as the first layer is composed of a plurality of tube-shaped resins different in flexibility. The other configurations are similar to those in the first embodiment and, therefore, the explanation thereof will not be provided.

That is, in Fig. 7A, reference numeral 30 denotes a flexible tube. The flexible tube 30 is primarily composed of a helical tube 13, a net-shaped tube 14 formed to have the shape of a net covering the helical tube 13, and an outer sheath resin 31 covering the outside of the net-shaped tube 14 in that order from the inside. The outer sheath resin 31 is formed into the shape of a taper at some midpoint of the flexible tube 30, while the taper has a wall thickness gradually increasing from the distal end side toward the base end side, as is clear from the appearance thereof.

The outer sheath resin 31 of the flexible tube 30 is composed of a plurality of layers, incidentally, is composed of two layers in the third embodiment. With respect to an outer sheath resin 31a, which is the first layer in the net-shaped tube 14 side of the outer sheath resin 31, the distal end side is formed of a soft tube resin 31al as a first resin, and the base end side is formed of a hard tube resin 31ah as a second resin. The end surface in the base end side of the soft tube resin 31al is formed to have a large inner diameter, and the end surface in the distal end side of the hard tube resin 31ah is formed to have a small outer diameter. In the outer sheath resin 31a serving as the first layer, the base end side of the soft tube resin 31al and the distal end side of the hard tube resin 31ah are engaged to constitute a engage portion 31as serving as a second flexibility-varying portion (a resin-hardness-varying portion).

In an outer sheath resin 31b serving as the second layer which is disposed outside the outer sheath resin 31a serving as the first layer of the outer sheath resin 31, a taper portion 31bt (a first flexibility-varying portion) is disposed in the distal end side while having a wall thickness gradually increasing from the start point located at a predetermined distance from the engage portion 31as of the outer sheath resin 31a serving as the first layer toward the base end side.

Here, for example, an ester-based resin is used as the resin material for the soft tube resin 31al of the outer sheath resin 31a serving as the first layer, and the same-ester-based but harder resin is used for the hard tube resin 31ah. For example, the same ester-based resin as that used for the soft tube resin 31al of the outer sheath resin 31a serving as the first layer is used as the resin material for the outer sheath resin 31b serving as the second layer. However, selection of these resin materials is not limited thereto, and resins of, for example, ester-based, olefin-based, styrene-based, and amide-based may be used alone or after blending for the outer sheath resins serving as respective layers in accordance with the required flexibility.

With respect to the thus prepared flexible tube 30 according to the third embodiment of the present invention, as in the above-described first embodiment, by dividing the manufacturing process into a step of ensuring the adhesion between the net-shaped tube 14 and the outer sheath resin 31a serving as the first layer and between the outer sheath resin 31a serving as the first layer and the outer sheath resin 31b serving as the second layer, a step of applying the coverings of the outer sheath resins 31a and 31b in which it is essential only that the outer diameters of the small diameter portion and the large diameter portion of the flexible tube 30 are satisfied, and a step of forming the taper portion 31bt in which it is essential only that the length and the start position of the taper portion 31bt of the outer sheath resin 31b serving as the second layer are satisfied, determination of each optimum condition becomes easy, and the flexible tube 30 can be manufactured optimally and easily.

Since no adhesive is required in the joining by the heat adhesion as in the above-described first embodiment, fluctuations in the adhesion due to variations in the adhesive are reduced. In particular, in the neighborhood of the start position of the taper portion 31bt, no adhesive extends off the portion where the outer sheath resin 31b serving as the second layer is disposed, nor occurs, conversely, shortage of the adhesive in the neighborhood of the start position of the taper portion 31bt

With respect to the flexible tube 30 according to the third embodiment of the present invention, as shown in Fig. 7B, initially, small flexibility variation is achieved by the engage portion 31as of the outer sheath resin 31a serving as the first layer. Subsequently, small stepwise flexibility variation is further achieved in between the engage portion 31as and the taper portion 31bt (a portion P in Fig. 7B). In the following taper portion 31bt, smooth flexibility variation is achieved (a portion Q in Fig. 7B). In this manner, in the entire flexible tube 30, the large flexibility variation can be achieved stepwise with a gentle, long gradient.

In the above-described flexible tube 30, a larger load is applied to the outer sheath resin 31b serving as the second layer when the flexible tube 30 is bended repeatedly. This is because larger distortion due to the bending occurs in the outer sheath resin 31b serving as the second layer located farther from the center line of the flexible tube 30. However, the bending resistance can be improved by using a soft resin for the outer sheath resin 31b serving as the second layer.

Further, various specifications are made for the desired flexible tube 30. However, the flexible tube 30 according to the third embodiment of the present invention can be flexibly complied with various specifications required for the flexibility variation by combining the shapes of the outer sheath resin 31a serving as the first layer, the outer sheath resin 31b serving as the second layer, and the taper portion 31bt, the length and the shape of the engage portion 31as of the outer sheath resin 31a serving as the first layer, and a compounding ratio of each resin.

In the flexible tube 30 according to the third embodiment of the present invention, a predetermined distance is provided between the engage portion 31as of the outer sheath resin 31a serving as the first layer and the taper portion 31bt of the outer sheath resin 31b serving as the second layer. However, the engage portion 31as and the taper portion 31bt may be disposed in a substantially continuous manner, or the two may be disposed in the locations overlapping with each other.

## Claims

1. A flexible tube of an endoscope, **characterized by** comprising:
a first flexibility-varying portion (15bt, 21bt, 31bt) disposed in an outer sheath (15, 21, 31) of an elongated flexible tube (10, 20, 30) having a distal end and a base end, wherein the first flexibility-varying portion (15bt, 21bt, 31bt) is disposed in order that the thickness of the outer sheath (15, 21, 31) in the distal end side of the first flexibility-varying portion (15bt, 21bt, 31bt) is varied from the thickness in the base end side and that the flexibility of the flexible tube (10, 20, 30) in the distal end side is varied from the flexibility in the base end side; and
a second flexibility-varying portion (15amix, 21bmix, 31as) disposed in the outer sheath (15, 21, 31), at a location different from that of the first flexibility-varying portion (15bt, 21bt, 31bt) on the distal end side of the first flexibility-varying portion (15bt, 21bt, 31bt) in a longitudinal direction of the flexible tube (10, 20, 30), wherein the second flexibility-varying portion (15amix, 21bmix, 31as) is disposed in order that the thickness of the outer sheath (15, 21, 31) is substantially not varied in the longitudinal direction of the flexible tube (10, 20, 30) and that that the flexibility of the flexible tube (10, 20, 30) in the distal end side is varied from the flexibility in the base end side.

2. The flexible tube of an endoscope according to Claim 1, **characterized in that** the outer sheath (15, 21, 31) is composed of a plurality of covering layers, and **characterized in that** the first flexibility-varying portion (15bt, 21bt, 31bt) is composed of a taper portion which is disposed in an outermost covering layer (15b, 21b, 31b) and which makes the thickness of the outermost covering (15b, 21b, 31b) increase from the distal end side toward the base end side.

3. The flexible tube of an endoscope according to Claim 1 or Claim 2, **characterized in that** the second flexibility-varying portion (15amix, 21bmix) is composed of a resin-hardness-varying portion in which a first resin (15al, 21b) is replaced with a second resin (15ah, 21bh) harder than the first resin (15al, 21bl) from the distal end side toward the base end side of the outer sheath (15a, 21b)

4. The flexible tube of an endoscope according to Claim 1 or Claim 2, **characterized in that** the resin-hardness-varying portion is formed by joining stepwise the first resin (31al) disposed in the shape of a tube in the distal end side and the second resin (31ah) disposed in the shape of a tube in the base end side to each other.

5. The flexible tube of an endoscope according to any one of Claim 1 to Claim 4, **characterized in that** the first flexibility-varying portion (15bt, 21bt, 31bt) and the second flexibility-varying portion (15amix, 21bmix, 31as) are disposed in order that the respective sections serving for varying the flexibility of the flexible tube (10, 20, 30) are arranged continuously.

6. The flexible tube of an endoscope according to any one of Claim 1 to Claim 4, **characterized in that** the first flexibility-varying portion (15bt, 21bt, 31bt) and the second flexibility-varying portion (15amix, 21bmix, 31as) are disposed in order that the respective sections serving for varying the flexibility of the flexible tube (10, 20, 30) are arranged with a predetermined distance therebetween.

7. The flexible tube of an endoscope according to any one of Claim 1 to Claim 4, **characterized in that** the first flexibility-varying portion (15bt, 21bt, 31bt) and the second flexibility-varying portion (15amix, 21bmix, 31as) are disposed to overlap with each other in a longitudinal direction of the outer sheath (15, 21, 31).

8. The flexible tube of an endoscope according to Claim 1, **characterized in that** the first flexibility-varying portion (15bt, 21bt, 31bt) and the second flexibility-varying portion (15amix, 21bmix, 31as) are disposed between the distal end and the base end of the flexible tube (10, 20, 30).

9. The flexible tube of an endoscope according to Claim 1, **characterized in that** the flexible tube (10, 20, 30) includes a third flexibility-non-varying portion and a fourth flexibility-non-varying portion of the same thickness and flexibility as the outer sheath (15, 21, 31), while the first flexibility-varying portion (15bt, 21bt, 31bt) and the second flexibility-varying portion (15amix, 21bmix, 31as) are disposed between the third flexibility-non-varying portion and the fourth flexibility-non-varying portion.

10. A method of manufacturing a flexible tube of an endoscope as defined in Claim 1, the method **characterized by** comprising the steps of:
forming a first covering layer (15a, 21a, 31a) on a member formed substantially in the shape of an elongated tube having a distal end and a base end, while the first covering layer (15a, 21a, 31a) includes a flexibility-varying portion (15amix, 21bmix, 31as) which varies the flexibility of the flexible tube (10, 20, 30) in a longitudinal direction substantially without varying the thickness;
forming a second covering layer (15b, 21b, 31b) on the outside of the first covering layer (15a, 21a, 31a), while the second covering layer (15b, 21b, 31b) includes a flexibility-varying portion (15bt, 21bt, 31bt) which varies the flexibility of the flexible tube (10, 20, 30) in the longitudinal direction by varying the thickness from the distal end side toward the base end side of the flexible tube (10, 20, 30); and
bringing the first covering layer (15a, 21a, 31a) and the second covering layer (15b, 21b, 31b) into intimate contact with each other, such that the flexibility-varying portion (15amix, 21bmix, 31as) of the first covering layer (15a, 21a31a) is on the distal end side of the flexibility-varying portion (15bt, 21bt, 31bt) of the second covering layer (15b, 21b, 31b).

11. The method of manufacturing a flexible tube of an endoscope according to Claim 10, **characterized in that** the flexibility-varying portion (15bt, 21bt, 31bt) of the second covering layer (15b, 21b, 31b) is formed by disposing a tapered portion in order that the thickness of the second covering layer (15b, 21b, 31b) is increased from the distal end side toward the base end side.

12. The method of manufacturing a flexible tube of an endoscope according to Claim 10 or Claim 11, **characterized in that** the second covering layer (31b) is disposed as an outermost portion of the member formed by joining the members substantially in the shape of a tube.

13. The method of manufacturing a flexible tube of an endoscope according to Claim 10 or Claim 11, **characterized in that** the flexibility-varying portion (15amix, 21mix) of the first covering layer (15a, 21a) is formed by replacing a first resin with a second resin harder than the first resin from the distal end side toward the base end side of the first covering layer (15a, 21a).

14. The method of manufacturing a flexible tube of an endoscope according to Claim 11, **characterized in that** the taper portion (15bt, 21bt, 31bt) is formed by grinding or fusing.

15. The method of manufacturing a flexible tube of an endoscope according to any one of Claim 10 to Claim 14, **characterized in that** the intimate contact between the first covering layer (15a, 21a, 31a) and the second covering layer (15b, 21b, 31b) is performed by heat adhesion.

## Patentansprüche

1. Ein flexibler Schlauch eines Endoskops, **dadurch gekennzeichnet, dass** er aufweist:
einen ersten Abschnitt (15bt, 21bt, 31bt) sich ändernder Flexibilität, der in einer äußeren Ummantelung (15, 21, 31) eines langgestreckten flexiblen Schlauchs (10, 20, 30) mit einem distalen Ende und einem Basisende angeordnet ist, wobei der erste Abschnitt (15bt, 21bt, 31bt) sich ändernder Flexibilität dafür angeordnet ist, dass sich die Dicke der äußeren Ummantelung (15, 21, 31) auf Seiten des distalen Endes des ersten Abschnitts (15bt, 21bt, 31bt) sich ändernder Flexibilität sich gegenüber der Dicke auf Seiten des Basisendes ändert und dass sich die Flexibilität des flexiblen Schlauchs (10, 20, 30) auf Seiten des distalen Endes gegenüber der Flexibilität von Seiten des Basisendes ändert; und
einen zweiten Abschnitt (15amix, 21bmix, 31as) sich ändernder Flexibilität, der in der äußeren Ummantelung (15, 21, 31) an einer Stelle unterschiedlich zu derjenigen des ersten Abschnitts (15bt, 21bt, 31bt) sich ändernder Flexibilität auf Seiten des distalen Endes des ersten Abschnitts (15bt, 21bt, 31bt) sich ändernder Flexibilität in Längsrichtung des flexiblen Schlauchs (10, 20, 30) angeordnet ist, wobei der zweite Abschnitt (15amix, 21bmix, 31as) sich ändernder Flexibilität dafür angeordnet ist, dass sich die Dicke der äußeren Ummantelung (15, 21, 31) in Längsrichtung des flexiblen Schlauchs (10, 20, 30) im wesentlichen nicht ändert und dass sich die Flexibilität des flexiblen Schlauchs (10, 20, 30) auf Seiten des distalen Endes gegenüber der Flexibilität auf Seiten des Basisendes ändert.

2. Der flexible Schlauch eines Endoskops nach Anspruch 1, **dadurch gekennzeichnet, dass** die äußere Ummantelung (15, 21, 31) aus einer Mehrzahl von Deckschichten aufgebaut ist und **dadurch gekennzeichnet ist, dass** der erste Abschnitt (15bt, 21bt, 31bt) sich ändernder Flexibilität aus einem sich verjüngenden Abschnitt aufgebaut ist, der in einer äußersten Deckschicht (15b, 21b, 31b) angeordnet ist und der bewirkt, dass sich die Dicke der äußersten Abdeckung (15b, 21b, 31b) von Seiten des distalen Endes her in Richtung zur Seite des Basisendes hin erhöht.

3. Der flexible Schlauch eines Endoskops nach Anspruch 1 oder Anspruch 2, **dadurch gekennzeichnet, dass** der zweite Abschnitt (15amix, 21bmix) sich ändernder Flexibilität aus einem Abschnitt sich ändernder Harzhärte aufgebaut ist, in welchem ein erstes Harz (15al, 21b) von der distalen Endseite in Richtung der Basisendseite der äußeren Ummantelung (15a, 21b) durch ein zweites Harz (15ah, 21bh) ersetzt ist, das härter als das erste Harz (15al, 21bl) ist.

4. Der flexible Schlauch eines Endoskops nach Anspruch 1 oder Anspruch 2, **dadurch gekennzeichnet, dass** der Abschnitt sich ändernder Harzhärte gebildet ist durch schrittweises Aneinanderfügen des ersten Harzes (31al), das in Form einer Röhre an der distalen Endseite angeordnet ist und des zweiten Harzes (31ah), das in Form einer Röhre an der Basisendseite angeordnet ist.

5. Der flexible Schlauch eines Endoskops nach einem der Ansprüche 1 bis 4, **dadurch gekennzeichnet, dass** der erste Abschnitt (15bt, 21bt, 31bt) sich ändernder Flexibilität und der zweite Abschnitt (15amix, 21bmix, 31as) sich ändernder Flexibilität so angeordnet sind, dass die jeweiligen Abschnitte, die zum Ändern der Flexibilität des flexiblen Schlauchs (10, 20, 30) dienen, durchgängig angeordnet sind.

6. Der flexible Schlauch eines Endoskops nach einem der Ansprüche 1 bis 4, **dadurch gekennzeichnet, dass** der erste Abschnitt (15bt, 21bt, 31bt) sich ändernder Flexibilität und der zweite Abschnitt (15amix, 21bmix, 31as) sich ändernder Flexibilität so angeordnet sind, dass die jeweiligen Abschnitte, die zum Ändern der Flexibilität des flexiblen Schlauchs (10, 20, 30) dienen, mit einem bestimmten Abstand zwischen sich angeordnet sind.

7. Der flexible Schlauch eines Endoskops nach einem der Ansprüche 1 bis 4, **dadurch gekennzeichnet, dass** der erste Abschnitt (15bt, 21bt, 31bt) sich ändernder Flexibilität und der zweite Abschnitt (15amix, 21bmix, 31as) sich ändernder Flexibilität so angeordnet sind, dass sie einander in Längsrichtung der äußeren Ummantelung (15, 21, 31) überlappen.

8. Der flexible Schlauch eines Endoskops nach Anspruch 1, **dadurch gekennzeichnet, dass** der erste Abschnitt (15bt, 21bt, 31bt) sich ändernder Flexibilität und der zweite Abschnitt (15amix, 21bmix, 31as) sich ändernder Flexibilität zwischen dem distalen Ende und dem Basisende des flexiblen Schlauchs (10, 20, 30) angeordnet sind.

9. Der flexible Schlauch eines Endoskops nach Anspruch 1, **dadurch gekennzeichnet, dass** der flexible Schlauch (10, 20, 30) einen dritten Abschnitt sich nicht ändernder Flexibilität und einen vierten Abschnitt sich nicht ändernder Flexibilität gleicher Dicke und Flexibilität wie die äußere Ummantelung (15, 21, 31) aufweist, wobei der erste Abschnitt (15bt, 21bt, 31bt) sich ändernder Flexibilität und der zweite Abschnitt (15amix, 21bmix, 31as) sich ändernder Flexibilität zwischen dem dritten Abschnitt sich nicht ändernder Flexibilität und dem vierten Abschnitt sich nicht ändernder Flexibilität angeordnet sind.

10. Ein Verfahren zur Herstellung eines flexiblen Schlauchs eines Endoskops, wie er in Anspruch 1 angegeben ist, wobei das Verfahren **gekennzeichnet ist durch** die folgenden Schritte:
Bilden einer ersten Deckschicht (15a, 21a, 31a) auf einem Bauteil, ausgebildet im wesentlichen in Form eines langgestreckten Schlauchs mit einem distalen Ende und einem Basisende, wobei die erste Deckschicht (15a, 21a, 31a) einen Abschnitt (15amix, 21bmix, 31as) sich ändernder Flexibilität enthält, der die Flexibilität des flexiblen Schlauchs (10, 20, 30) in Längsrichtung ändert, ohne die Dicke wesentlich zu ändern;
Bilden einer zweiten Deckschicht (15b, 21b, 31b) auf der Außenseite der ersten Deckschicht (15a, 21a, 31a), wobei die zweite Deckschicht (15b, 21b, 31b) einen Abschnitt (15bt, 21bt, 31bt) sich ändernder Flexibilität enthält, der die Flexibilität des flexiblen Schlauchs (10, 20, 30) in Längsrichtung ändert, indem die Dicke von der Seite des distalen Endes her in Richtung der Seite des Basisendes des flexiblen Schlauchs (10, 20, 30) geändert wird; und
Bringen der ersten Deckschicht (15a, 21a, 31a) und der zweiten Deckschicht (15b, 21b, 31b) in engen Kontakt miteinander, so dass der Abschnitt (15amix, 21bmix, 31as) sich ändernder Flexibilität der ersten Deckschicht (15a, 21a, 31a) an der distalen Endseite des Abschnitts (15bt, 21bt, 31bt) sich ändernder Flexibilität der zweiten Deckschicht (15b, 21b, 31b) ist.

11. Das Verfahren zur Herstellung eines flexiblen Schlauchs eines Endoskops nach Anspruch 10, **dadurch gekennzeichnet, dass** der Abschnitt (15bt, 21bt, 31bt) sich ändernder Flexibilität der zweiten Deckschicht (15b, 21b, 31b) gebildet wird durch Anordnen eines sich verjüngenden Abschnitts, so dass sich die Dicke der zweiten Deckschicht (15b, 21b, 31b) von der Seite des distalen Endes her in Richtung der Seite des Basisendes erhöht.

12. Das Verfahren zur Herstellung eines flexiblen Schlauchs eines Endoskops nach Anspruch 10 oder Anspruch 11, **dadurch gekennzeichnet, dass** die zweite Deckschicht (31b) als ein äußerster Abschnitt des Bauteils angeordnet ist, das gebildet wird durch Aneinanderfügen der Bauteile im wesentlichen in Form einer Röhre.

13. Das Verfahren zur Herstellung eines flexiblen Schlauchs eines Endoskops nach Anspruch 10 oder 11, **dadurch gekennzeichnet, dass** der Abschnitt (15amix, 21bmix) sich ändernder Flexibilität der ersten Deckschicht (15a, 21a) gebildet wird durch Ersetzen eines ersten Harzes durch ein zweites Harz, das härter als das erste Harz ist, von der Seite des distalen Endes her in Richtung der Basisendseite der ersten Deckschicht (15a, 21a).

14. Das Verfahren zur Herstellung eines flexiblen Schlauchs eines Endoskops nach Anspruch 11, **dadurch gekennzeichnet, dass** der sich verjüngende Abschnitt (15bt, 21bt, 31bt) durch Schleifen oder Abschmelzen gebildet wird.

15. Das Verfahren zur Herstellung eines flexiblen Schlauchs eines Endoskops nach einem der Ansprüche 10 bis 14, **dadurch gekennzeichnet, dass** der enge Kontakt zwischen der ersten Deckschicht (15a, 21a, 31a) und der zweiten Deckschicht (15b, 21b, 31b) durch Wärmeanhaftung durchgeführt wird.

## Revendications

1. Tube flexible d'un endoscope, **caractérisé en ce qu'**il comprend :
une première partie à flexibilité variable (15bt, 21bt, 31bt) disposée dans une gaine extérieure (15, 21, 31) d'un tube flexible allongé (10, 20, 30) ayant une extrémité distale et une extrémité de base, dans laquelle la première partie à flexibilité variable (15bt, 21bt, 31bt) est disposée afin que l'épaisseur de la gaine extérieure (15, 21, 31) du côté de l'extrémité distale de la première partie à flexibilité variable (15bt, 21bt, 31bt) varie par rapport à l'épaisseur du côté de l'extrémité de base et que la flexibilité du tube flexible (10, 20, 30) du côté de l'extrémité distale varie par rapport à la flexibilité du côté de l'extrémité de base ; et
une deuxième partie à flexibilité variable (15amix, 21bmix, 31as) disposée dans la gaine extérieure (15, 21, 31), au niveau d'un emplacement différent de celui de la première partie à flexibilité variable (15bt, 21bt, 31bt) du côté de l'extrémité distale de la première partie à flexibilité variable (15bt, 21bt, 31bt) dans une direction longitudinale du tube flexible (10, 20, 30), dans laquelle la deuxième partie à flexibilité variable (15amix, 21bmix, 31as) est disposée afin que l'épaisseur de la gaine extérieure (15, 21, 31) ne varie sensiblement pas dans la direction longitudinale du tube flexible (10, 20, 30) et que la flexibilité du tube flexible (10, 20, 30) du côté de l'extrémité distale varie par rapport à la flexibilité du côté de l'extrémité de base.

2. Tube flexible d'un endoscope selon la revendication 1, **caractérisé en ce que** la gaine extérieure (15, 21, 31) est composée d'une pluralité de couches de couverture, et **caractérisé en ce que** la première partie à flexibilité variable (15bt, 21bt, 31bt) est composée d'une partie resserrée qui est disposée dans une couche de couverture la plus à l'extérieur (15b, 21b, 31b) et qui fait augmenter l'épaisseur de la couverture la plus à l'extérieur (15b, 21b, 31b) depuis le côté de l'extrémité distale vers le côté de l'extrémité de base.

3. Tube flexible d'un endoscope selon la revendication 1 ou la revendication 2, **caractérisé en ce que** la deuxième partie à flexibilité variable (15amix, 21bmix) est composée d'une partie en résine de dureté variable dans laquelle une première résine (15al, 21b) est remplacée par une seconde résine (15ah, 21bh) plus dure que la première résine (15al, 21bl) depuis le côté de l'extrémité distale vers le côté de l'extrémité de base de la gaine extérieure (15a, 21b).

4. Tube flexible d'un endoscope selon la revendication 1 ou la revendication 2, **caractérisé en ce que** la partie en résine de dureté variable est formée en joignant progressivement la première résine (31al) disposée sous la forme d'un tube du côté de l'extrémité distale et la seconde résine (31ah) disposée sous la forme d'un tube du côté de l'extrémité de base l'une à l'autre.

5. Tube flexible d'un endoscope selon l'une quelconque des revendications 1 à 4, **caractérisé en ce que** la première partie à flexibilité variable (15bt, 21bt, 31bt) et la deuxième partie à flexibilité variable (15amix, 21bmix, 31as) sont disposées afin que les sections respectives servant à faire varier la flexibilité du tube flexible (10, 20, 30) soient agencées de façon continue.

6. Tube flexible d'un endoscope selon l'une quelconque des revendications 1 à 4, **caractérisé en ce que** la première partie à flexibilité variable (15bt, 21bt, 31bt) et la deuxième partie à flexibilité variable (15amix, 21bmix, 31as) sont disposées afin que les sections respectives servant à faire varier la flexibilité du tube flexible (10, 20, 30) soient agencées avec une distance prédéterminée entre elles.

7. Tube flexible d'un endoscope selon l'une quelconque des revendications 1 à 4, **caractérisé en ce que** la première partie à flexibilité variable (15bt, 21bt, 31bt) et la deuxième partie à flexibilité variable (15amix, 21bmix, 31as) sont disposées pour se chevaucher l'une l'autre dans une direction longitudinale de la gaine extérieure (15, 21, 31).

8. Tube flexible d'un endoscope selon la revendication 1, **caractérisé en ce que** la première partie à flexibilité variable (15bt, 21bt, 31bt) et la deuxième partie à flexibilité variable (15amix, 21bmix, 31as) sont disposées entre l'extrémité distale et l'extrémité de base du tube flexible (10, 20, 30).

9. Tube flexible d'un endoscope selon la revendication 1, **caractérisé en ce que** le tube flexible (10, 20, 30) comprend une troisième partie à flexibilité non variable et une quatrième partie à flexibilité non variable de la même épaisseur et flexibilité que la gaine extérieure (15, 21, 31), tandis que la première partie à flexibilité variable (15bt, 21bt, 31bt) et la deuxième partie à flexibilité variable (15amix, 21bmix, 31as) sont disposées entre la troisième partie à flexibilité non variable et la quatrième partie à flexibilité non variable.

10. Procédé de fabrication d'un tube flexible d'un endoscope selon la revendication 1, le procédé étant **caractérisé en ce qu'**il comprend les étapes consistant à :
former une première couche de couverture (15a, 21a, 31a) sur un élément ayant sensiblement la forme d'un tube allongé ayant une extrémité distale et une extrémité de base, tandis que la première couche de couverture (15a, 21a, 31a) comprend une partie à flexibilité variable (15amix, 21bmix, 31as) qui fait varier la flexibilité du tube flexible (10, 20, 30) dans une direction longitudinale sensiblement sans faire varier l'épaisseur ;
former une seconde couche de couverture (15b, 21b, 31b) sur l'extérieur de la première couche de couverture (15a, 21a, 31a), tandis que la seconde couche de couverture (15b, 21b, 31b) comprend une partie à flexibilité variable (15bt, 21bt, 31bt) qui fait varier la flexibilité du tube flexible (10, 20, 30) dans la direction longitudinale en faisant varier l'épaisseur depuis le côté de l'extrémité distale vers le côté de l'extrémité de base du tube flexible (10, 20, 30) ; et
mettre la première couche de couverture (15a, 21a, 31a) et la seconde couche de couverture (15b, 21b, 31b) en contact étroit l'une avec l'autre, de manière à ce que la partie à flexibilité variable (15amix, 21bmix, 31as) de la première couche de couverture (15a, 21a, 31a) soit sur le côté de l'extrémité distale de la partie à flexibilité variable (15bt, 21bt, 31bt) de la seconde couche de couverture (15b, 21b, 31b).

11. Procédé de fabrication d'un tube flexible d'un endoscope selon la revendication 10, **caractérisé en ce que** la partie à flexibilité variable (15bt, 21bt, 31bt) de la seconde couche de couverture (15b, 21b, 31b) est formée en disposant une partie resserrée afin que l'épaisseur de la seconde couche de couverture (15b, 21b, 31b) augmente depuis le côté de l'extrémité distale vers le côté de l'extrémité de base.

12. Procédé de fabrication d'un tube flexible d'un endoscope selon la revendication 10 ou la revendication 11, **caractérisé en ce que** la seconde couche de couverture (31b) est disposée comme une partie la plus à l'extérieur de l'élément formé en joignant les éléments sensiblement sous la forme d'un tube.

13. Procédé de fabrication d'un tube flexible d'un endoscope selon la revendication 10 ou la revendication 11, **caractérisé en ce que** la partie à flexibilité variable (15amix, 21bmix) de la première couche de couverture (15a, 21a) est formée en remplaçant une première résine par une seconde résine plus dure que la première résine depuis le côté de l'extrémité distale vers le côté de l'extrémité de base de la première couche de couverture (15a, 21a).

14. Procédé de fabrication d'un tube flexible d'un endoscope selon la revendication 11, **caractérisé en ce que** la partie resserrée (15bt, 21bt, 31bt) est formée par broyage ou par fusion.

15. Procédé de fabrication d'un tube flexible d'un endoscope selon l'une quelconque des revendications 10 à 14, **caractérisé en ce que** le contact étroit entre la première couche de couverture (15a, 21a, 31a) et la seconde couche de couverture (15b, 21b, 31b) est réalisé par adhérence par la chaleur.
